# EUROPÄISCHE PATENTSCHRIFT

(11) **EP 2 481 708 B1**
(45) Veröffentlichungstag und Bekanntmachung des Hinweises auf die Patenterteilung: **10.07.2013**
(21) Anmeldenummer: 12153198.2
(22) Anmeldetag: 31.01.2012
(51) Int. Cl.: C01B 33/107, B01D 3/14

(54) **Verfahren zur destillativen Reinigung von Chlorsilanen**
Process for purifying chlorosilanes by distillation
Procédé de nettoyage par distillation de chlorosilanes

(30) Priorität: 01.02.2011 DE 102011003453
(43) Veröffentlichungstag der Anmeldung: 01.08.2012
(73) Patentinhaber: Wacker Chemie AG, 81737 München (DE)
(72) Erfinder: Pätzold, Dr. Uwe, 84489 Burghausen (DE); Häckl, Dr. Walter, 84367 Zeilarn (DE); Prochaska, Jan, 84547 Emmerting (DE)
(74) Vertreter: Killinger, Andreas

(56) Entgegenhaltungen:
- DD-A3- 158 322
- DE-A1-102007 014 107
- DE-A1-102008 002 537

## Beschreibung

Die vorliegende Erfindung betrifft ein Verfahren zur destillativen Reinigung von Chlorsilanen.

Die Herstellung von polykristallinem Silicium, welches z.B. in der Photovoltaik oder in der Halbleiter-Industrie Verwendung findet, geht vom Rohstoff Trichlorsilan (TCS) aus.

Dieses TCS wird hauptsächlich über drei verschiedene Verfahren erzeugt.
**A)** Si + 3 HCl → SiHCl₃ + H₂ + Nebenprodukte
**B)** Si + 3 SiCl₄ + 2 H₂ → 4 SiHCl₃ + Nebenprodukte
**C)** SiCl₄ + H₂ → SiHCl₃ + HCl + Nebenprodukte

Bei diesen Prozessen entstehen neben anderen Nebenprodukten oder Verunreinigungen größere Mengen an Dichlorsilan (DCS).

So ist bekannt, dass bei der Hydrochlorierung von metallurgischem Silicium gemäß **(A)** etwa 0,1-1,0 % DCS im Reaktionsprodukt enthalten sind.

Die Umsetzung von metallurgischem Silicum mit Siliciumtetrachlorid (STC) und Wasserstoff gemäß **(B)** liefert in der Regel noch höhere DCS-Gehalte im Reaktionsprodukt, insbesondere wenn Kupfer als Katalysator für diesen Prozess verwendet wird.

Auch in der Hydrierung von STC gemäß **(C)** findet man im Reaktionsprodukt 0,05-1,0 % DCS.

DCS ist an sich ein nützliches Produkt, welches in der Halbleiter-Industrie zur Abscheidung von Silicium, aber auch zur Herstellung von organofunktionellen Silanen Verwendung finden könnte.

Dies setzt allerdings eine sehr hohe Reinheit voraus. Beispielsweise sollte für Halbleiteranwendungen die Konzentration von Bor < 10 ppta betragen.

Als weiteres Beispiel sei die Hydrosilylierung genannt. Bei der Hydrosilylierung werden Derivate von Hydrosilanen durch eine katalytische Additionsreaktion an Vinylgruppen oder anderen Mehrfachbindungen umgesetzt. Typische Katalysatoren sind Komplexe des Edelmetalls Platin. Hier sollte die Konzentration von Bor < 1 ppbw betragen, da Bor als Katalysatorgift wirkt.

DCS aus den zuvor genannten Prozessen **A-C** ist für diese Anwendungen ungeeignet, da besonderes die Bor-Gehalte zu hoch sind.

Da Bor hauptsächlich als BCl₃ mit einem Siedepunkt von 8,3°C vorliegt und einen ähnlichen Siedepunkt wie DCS (Siedepunkt 12,5°C) aufweist, konzentriert sich Bor in der nachfolgenden Destillation fast vollständig im DCS-Produktstrom auf.

Trotz eines Unterschieds im Siedepunkt von knapp 30 K gelingt eine destillative Abtrennung von BCl₃ aus TCS nicht vollständig, insbesondere wenn man Bor-Gehalte von < 0,1 ppm im TCS erreichen will.

Im Stand der Technik werden die bei der Hydrochlorierung von metallurgischem Silicium erzeugten Mengen an BCl₃ zusammen mit einer Menge Trichlorsilan aus dem System ausgeschleust. Dies ist z.B. dargestellt in "Handbook of Semiconductor Silicon Technology", William C. O'Mara, Robert B. Herring and Lee P. Hunt, Noyes Publications, USA 1990, siehe Seite 4, Fig. 2.

Wegen des sehr ähnlichen Siedepunkts wird zusammen mit BCl₃ auch DCS aus dem System ausgeschleust, was zu einer geringeren Wirtschaftlichkeit der Gesamtanlage führt.

Für die Abtrennung von Bor-Verunreinigungen aus TCS sind im Wesentlichen vier verschiedene Ansätze bekannt.

So wurden rein destillative Verfahren, aber auch Verfahren mit einem Hydrolyse-, Komplexierungs- oder einem Adsorptionsschritt beschrieben.

In DE 10 2007 014 107 A1 wird ein Verfahren zur Gewinnung borabgereicherter Chlorsilane aus einer borhaltigen Chlorsilanmischung durch destillative Abtrennung eines borangereicherten Destillationsstroms beschrieben, wobei in einer Anordnung von ein oder mehreren Destillationskolonnen zumindest bei einer Destillationskolonne ein borangereicherter Seitenstrom abgezweigt und entsorgt oder einer sonstigen Verwendung zugeführt wird. Mittels unterschiedlicher Kolonnenschaltungen und Produktentnahmen aus Kopf- bzw. Blasenseitenabzügen der jeweiligen Kolonnen kann dabei in Teilströmen der Bor-Gehalt im reinen DCS bis etwa 50 ppm abgereichert werden. Allerdings wird Bor in einem anderen Teilstrom, der DCS und TCS enthält, noch stärker angereichert. Ein weiterer Nachteil besteht darin, dass eine nicht unerhebliche Menge an TCS als Abfall verloren geht.

DE 10 2008 002 537 A1 offenbart ein Verfahren zur Verminderung des Gehaltes an Bor in Zusammensetzungen I umfassend mindestens ein Siliziumhalogenid, indem in einem ersten Schritt die Zusammensetzung I mit bis zu 600 mg Feuchte je Kilogramm der Zusammensetzung I in Kontakt gebracht wird, gegebenenfalls wird die mit Feuchte in Kontakt gebrachte Zusammensetzung I des ersten Schrittes mindestens einmal vollständig oder teilweise einem Teilschritt zur Abtrennung hydrolysierter Bor und/oder Silizium enthaltenden Verbindungen zugeführt und eine vorgereinigte Zusammensetzung II erhalten, die vollständig oder teilweise erneut dem ersten Schritt oder einem zweiten Schritt des Verfahrens zugeführt wird, und wobei in dem zweiten Schritt hydrolysierte Bor und/oder Silizium enthaltende Verbindungen destillativ abgetrennt werden, indem als Destillat eine vorgereinigte Zusammensetzung II mit einem verminderten Gehalt an Bor erhalten wird.

Der Bor-Gehalt in Chlorsilanen lässt sich also verringern, indem man z.B. Wasser in einer geeigneten Form zugibt. Durch Reaktion von Bor-Halogenid mit Wasser entstehen dabei höher siedende Hydrolysate, die sich destillativ leichter von Chlorsilan abtrennen lassen. Diese Verfahren benötigen allerdings einen zusätzlichen Ausschleusestrom, um die entstandenen Bor- und Chlorsilanhydrolysate abzutrennen (z.B. > 5% Ausschleusemenge bezogen auf das Einsatzprodukt). Problematisch sind auch Ablagerungen von Kieselsäure in Anlagenteilen und Korrosion durch dabei gebildetes HCl. Die Korrosion führt in der Folge zur Freisetzung von Dotierstoffen wie P und As aus dem Stahl der Anlagen.

In EP 2 036 858 A2 wird ein Verfahren beansprucht, in dem Bor-und Phosphor-haltige Chlorsilane mit dem Komplexbildner Benzaldehyd und Sauerstoff in Kontakt gebracht werden. Durch Oxidation und Komplexbildung können die im Chlorsilan enthaltenen Bor-Verbindungen leicht abgetrennt werden. Wie im Beispiel 6 dieser Anmeldung beschrieben, fallen dabei allerdings ca. 10% Rückstände an, mit denen der Bor-Komplex ausgeschleust werden muss. Auf Grund der relativ langsamen Reaktion (30 min) ist dieses Verfahren nicht für eine kontinuierliche Fahrweise geeignet. Zusätzlich erhöht sich der apparative Aufwand durch einen Rührkessel und der Eintrag von organischen Kontaminationen in das Zielprodukt ist wahrscheinlich.

In DE 10 2008 054 537 wird ein Verfahren zur Behandlung einer Zusammensetzung enthaltend mindestens eine Siliciumverbindung sowie mindestens ein Fremdmetall und/oder eine Fremdmetall enthaltende Verbindung beschrieben, wobei die Zusammensetzung in einem ersten Schritt mit mindestens einem Adsorptionsmittel und/oder mindestens einem ersten Filter in Kontakt gebracht wird, und gegebenenfalls in einem weiteren Schritt mit mindestens einem Filter in Kontakt gebracht wird, wobei eine Zusammensetzung gewonnen wird, in der der Gehalt des Fremdmetalls und/oder der Fremdmetall enthaltenden Verbindung vermindert ist.

Hier wird der Bor-Gehalt in Chlorsilanen durch Inkontaktbringen mit wasserfreien Adsorbermittel abgesenkt. Allerdings werden sehr große Mengen an Asdorbermittel (120 g/ 250 ml TCS) benötigt, um den gewünschten Reinigungszweck zu erzielen. Dies macht das Verfahren unwirtschaftlich, zumal ein kontinuierlicher Prozess kaum möglich ist, was bei der Hewgtellung von Chlorsilanen in Halbleiter-Qualität wirtschaftlich nachteilig ist. Der Einsatz von Adsorbern erfordert zudem weiteren apparativen Aufwand (wie Filtration) und beinhaltet das Risiko zum Eintrag anderer Verunreinigungen in das Halbleiter-reine Produkt.

Aus der beschriebenen Problomtik ergab sich die Aufgabenstellung der Erfindung, verunreinigte Chlorsilane mit gerinmgem Aufwand zu reinigen und dabei die vorhandenen Verunreinigungen in möglichst kleinen Ausschleusemengen anzureichern und abzuführen. Im heutigen wirtschaftlichen Maßstab muss die Ausbeute an TCS deutlich mehr 95% betragen.

Es hat sich gezeigt, dass rein destillative Verfahren von vorteil sind, da kein zusätzlicher apparativer Aufwand nötig wird und diese Verfahren auf einfach weise kontinuierlich betrieben werden können. Die Verluste an Chlorsilanen können dabei am besten minimiert werden.

Verteilhaft an den destillativen Verfahren ist die Tatsache, dass das Risiko eines Eintrags weiterer verunreinigungnen sehr gering ist.

Die Aufgabe der Erfindung wird gelöst durch ein Verfahren zur destillativen Reinigung von Chlorsilanen, umfassend Bereitstellen einer borhaltigen Mischung aus Chlorsilanen enthaltend TCS, DCS und STC und destillative Reinigung der Mischung aus Chlorsilanen in mehreren Destillationskolonnen, dadurch gekennzeichnet, dass die bereitgestellte Mischung aus Chlorsilanen einer Trennkolonne (2) zugeführt wird, deren Kolonnenparameter so gewählt werden, dass in einer ersten Fraktion (3) aus Trennkolonne (2) weniger als 10 ppm STC und in einer zweiten Fraktion (4) aus Trennkolonne (2) weniger als 10 ppm TCS enthalten sind, wobei Fraktion (3) aus Trennkolonne (2) einer dritten Kolonne (6) zugeführt und destillativ in einen Sumpfstrom (6b), der TCS enthält, und einen borangereicherten Kopfstrom (6a), der neben TCS Niedersieder wie DCS enthält, getrennt wird und Fraktion (4) aus Trennkolonne (2) einer zweiten Kolonne (5) zugeführt und destillativ in einen Kopfstrom enthaltend STC (5a) und einen borangereicherten Sumpfstrom enthaltend Hochsieder (5b) getrennt wird, um niedrig-siedende Borverbindungen mittels Kopfströmen enthaltend borangereichertes DCS und höher-siedende Borverbindungen mittels eines borangereicherten Sumpfstromes enthaltend Hochsieder aus den Destillationskolonnen abzuweigen.

Vorzugsweise wird die bereit gestellte Mischung aus Chlorsilanen mittels Reaktion von metallurgischem Silicium mit HCl in einem Wirbelschichtreaktor bei 350-400 °C erzeugt.

Die bereitgestellte Mischung aus Chlorsilanen wird einer Trennkolonne zugeführt, deren Kolonnenparameter so gewählt werden, dass in einer ersten Fraktion aus dieser Trennkolonne weniger als 10 ppm STC und in einer zweiten Fraktion aus dieser Trennkolonne weniger als 10 ppm TCS enthalten sind.

Entsprechende Kolonnenparameter sind u.a. Druck, Sumpftemperatur und Zahl der Trennstufen.

Zudem wird die zweite Fraktion aus der Trennkolonne einer zweiten Kolonne zugeführt und destillativ in einen Kopfstrom enthaltend STC und einen borangereicherten Sumpfstrom enthaltend Hochsieder getrennt.

Die erste Fraktion aus der Trennkolonne wird einer dritten Kolonne zugeführt und destillativ in einen Sumpfstrom, der TCS enthält, und einen borangereicherten Kopfstrom, der neben TCS Niedersieder wie DCS enthält, getrennt.

Der Kopfstrom enthaltend TCS und Niedersieder wie DCS aus der dritten Kolonne wird in eine vierte Kolonne geführt, wobei Inertgas eingespeist wird, wobei ein Kopfstrom aus der vierten Kolonne enthaltend borangereichertes DCS ausgeschleust wird, ein Sumpfstrom aus der vierten Kolonne der Trennkolonne wieder zugeführt wird und ein Nebenstrom enthaltend Abgas aus der vierten Kolonne entsorgt wird.

Jene vierte Kolonne wird vorzugsweise in Überdruck betrieben.

Vorzugsweise wird der Kopfstrom aus der dritten Kolonne enthaltend TCS und Niedersieder wie DCS vor Zuführen in die vierte Kolonne verflüssigt.

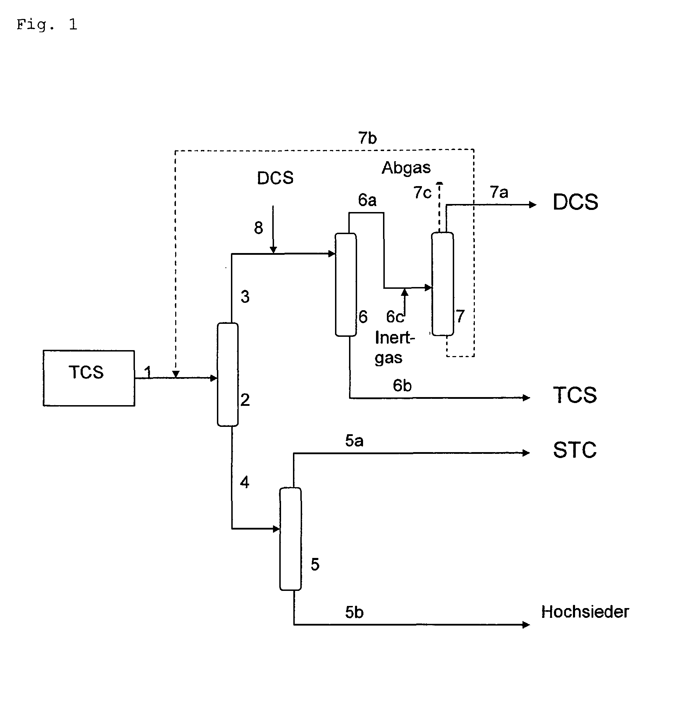

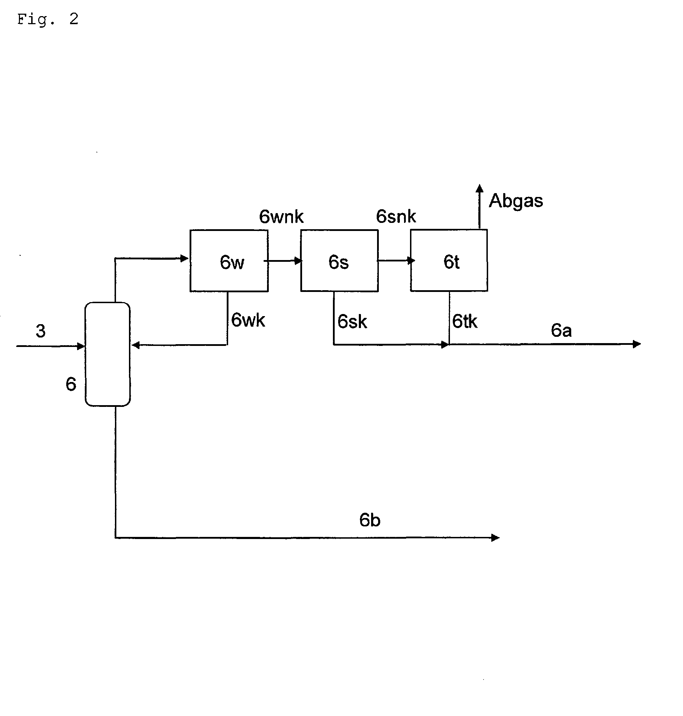

Die Erfindung wird im Folgende anhand von **Fig. 1** und **2** erläutert.
**Fig. 1** zeigt ein Flussdiagramm eines Verfahrens zur destillativen Aufarbeitung einer Chlorsilan-Mischung.
**Fig. 2** zeigt schematisch die Kondensation des Koptproduktes einer Destillationskolonne.

**Fig. 1** zeigt das Prinzip der destillativen Aufarbeitung einer Chlorsilan-Michung, die als Reaktionsprodukt der Hydrochlorierung von mg-Silicium anfällt. Das wesentliche Ziel ist dabei die Abtrennung von Bor- und Phosphorverunreinigungen aus dem Zielprodukt TCS.

**Fig. 2** zeigt die Kondensation des Kopfproduktee der Destillationskolonne **6**, vgl. **Fig. 1**.

Das Kopfprodukt wird nacheinander mit einem Wasserkühler **6w**, einem Solekühler **6s** und einem Tieftemperaturkühler (Frigen) 6t abgekühlt. Das jeweils entstehende Kondensat wird wiederverwertet. Das Kondensat des Wasserkühlers wird in die Kolonne zurückgeführt. Die Kondensate des Sole- und Tieftemperaturkühlers werden als Produktstrom 6a zur Kolonne 7 geführt. Das Abgas wird entsorgt.

Die Erfindung basiert auf umfangreichen analytischen Untersuchungen zur Verteilung der Bor-Verunreinigungen in den verschiedenen Chlorsilan-Fraktionen eines Verbundes zur Herstellung von polykristallinem Silicium.

Die wesentlichen Schritte der vorliegenden Erfindung sind die Erzeugung von Chlorsilanen, bevorzugt TCS, durch Hydrochloriderung von metallurgischem Silicium, die destillative Aufreinigung der Chlorsilane und die Abtrennung von stark mit Bor verunreinigten DCS- und STC-Fraktionen aus dieser Mischung aus Chlorsilanen.

Eine effektive Bor-Abtrennung von TCS aus der Hydrochlorierung von mg-Silicium lässt sich erfindungsgemäß destillativ so erzielen, indem eine im Folgenden beschriebene Verschaltung verschiedener Kolonnen verwendet wird.

Ziel ist es, die höher siedenden Bor-Verunreinigungen in einen STC-Teilstrom und niedrig siedenden Bor-Verbindungen in einem DCS-Teilstrom aufzukonzentrieren.

Dabei lässt sich TCS erzeugen, das weniger als 20 ppb Bor enthält bei gleichzeitiger weitestgehender Vermeidung von TCS im Abfall.

Das durch die Reaktion von handelsüblichem metallurgischen Silicium mit HCl in einem Wirbelschichtreaktor bei 350-400 °C erhaltene Gemisch aus Chlorsilanen **1 (****Fig. 1****)** enthaltend 86% TCS, 13,5 % STC, 0,3% DCS, 3,2 ppm Bor, sowie Spuren weiterer Verunreinigungen (Methylchlorsilane, Kohlenwasserstoffe, Hochsieder, wie z.B. Siloxane und Disilane) wird einer Trennkolonne **2** zugeführt.

Dabei werden die Kolonnenparameter so gewählt, dass im Kopfprodukt **3** weniger als 10 ppm STC und im Blasenprodukt **4** weniger als 10 ppm TCS enthalten sind.

Das Blasenprodukt **4** wird auf eine weitere Kolonne **5** geführt und dort in eine Fraktion STC **5a** und eine Fraktion Hochsieder **5b** (wie Siloxane, Disilane, Methyltrichlorsilan und ggf. Metallchloride) getrennt.

Die hochsiedenden Verbindungen **5b** können kontinuierlich oder batchweise aus der Kolonnenblase abgetrennt werden, da sie nur etwa 1% der Gesamtmenge ausmachen.

Das Kopfprodukt **3** der Kolonne **2** wird in einer nächsten Kolonne **6** in eine Fraktion **6b,** die sauberes TCS enthält, und eine Fraktion **6a** getrennt, die neben TCS noch Niedersieder enthält.

Zum Kopfprodukt **3** aus Kolonne **2** können vor Kolonne **6** noch zusätzliche Ströme an verunreinigtem DCS, das TCS enthält, eingeschleust werden, insofern sie nur vernachlässigbar kleine Mengen an niedriger siedender Komponenten als TCS enthalten.

Die Fraktion **6b** steht für die weitere Verarbeitung zur Verfügung.

Die Fraktion **6a** enthält neben DCS noch nicht unerhebliche Mengen an TCS und niedrig siedende Verunreinigungen wie zum Beispiel BCl₃.

Diese Fraktion wird zu einer zusätzlichen Kolonne **7** geführt, wobei in einer besonders bevorzugten Variante zusätzlich Inert-gas eingespeist werden kann. Kolonne **7** ist technisch so ausgeführt, dass sie in Überdruck betrieben werden kann.

Das Sumpfprodukt **7b** aus Kolonne **7** wird wieder zurückgeführt zur Verwendung in Kolonne **2.**

Das Abgas **7c** aus Kolonne **7,** das erhebliche Mengen an Bor enthält, kann über einen Wäscher der weiteren Entsorgung zugeführt werden.

Das Kopfprodukt **7a** aus Kolonne **7** enthält neben DCS einen Großteil der Verunreinigung an Bor.

Dieser Strom dient daher zur effektiven zusätzlichen Ausschleusung der Verunreinigung an Bor aus dem System.

Wie später in den Beispielen noch gezeigt, erhält man überraschenderweise eine drastische Reduzierung des Gehaltes an Bor im TCS Strom, wenn das Kopfprodukt aus Kolonne **6** mit einer mehrstufigen Kühlung verflüssigt wird und das Kondensat der jeweiligen Kühlschritte geeignet geführt wird. (siehe **Fig. 2****).** Als besonders geeignet hat es sich erwiesen, das Kopfprodukt der Kolonne **6** zunächst mit einem Wasserkühler **6w** auf eine Temperatur von etwa 10-30 °C, bevorzugt 15-25 °C zu kühlen.

Das Kondensat **6wk** dieses Kühlers wird in die Kolonne zurückgeführt.

Die nicht kondensierten Anteile **6wnk** werden einem Solekühler **6s** zugeführt, der den Produktstrom auf etwa -7 °C kühlt.

Das Kondensat **6sk** aus diesem Solekühler bildet die erste Komponente des Stromes **6a.**

Die im Solekühler nicht kondensierten Anteile **6snk** werden einer Tieftemperaturkühlung **6t** zugeführt und dort zu **6tk** kondensiert.

Sie bilden die zweite Komponente für **6a.** Die Tieftemperaturkühlung kühlt den Produktstrom auf etwa -60°C ab. Die dort wiederum nicht kondensierten Anteile werden als Abgas entsorgt. Der Gesamtproduktstrom **6a** wird zur Kolonne 7 geführt.

Der Teilstrom **6b** des beschriebenen Verfahrens ist das Zielprodukt, aufgereinigtes TCS, eines Chlorsilan-Verbundes zur Herstellung von Poly-Silicium.

Das auf diese Weise hergestellte TCS kann direkt oder in Mischung mit anderen Chlorsilan-Strömen zur Abscheidung von Poly-silicium in Solar-Qualität verwendet werden oder durch weitere destillative Schritte bis zur Halbleiter-Qualität aufgereinigt werden.

### Beispiele

Das durch die Reaktion von handelsüblichem metallurgischem Silicium (Bor-Gehalt 32 ppm) mit Chlorwasserstoff-Gas in einem Wirbelschichtreaktor bei 350-400 °C erhaltene Chlorsilangemisch **1** mit der Zusammensetzung von 86% TCS, 13,5 % STC, 0,3% DCS, 3,2 ppm Bor, sowie Spuren weiterer Verunreinigungen (Methylchlorsilane, Kohlenwasserstoffe, Hochsieder, wie z.B. Siloxane und Disilane) wurde destillativ aufgearbeitet.

Der Kopfstrom **3** der Kolonne **2** enthielt 3,4 ppm Bor (hauptsächlich leicht flüchtiges BCl₃), der Blasenstrom **4** enthielt 1,1 ppm schwerer siedender Bor-Verbindungen.

Der STC-Strom **4** wurde in Kolonne **5** aufdestilliert, über den Blasenstrom wurden die Hochsieder abgetrennt, die Abtrennung der Bor-Verbindungen gelingt dabei nicht vollständig, da der Kopfstrom **5a** immer noch 1 ppm Bor enthielt.

### Vergleichsbeispiel 1

Der Chlorsilanstrom **3** wurde in einer nachfolgenden Kolonne **6** aufdestilliert. Dies erfolgte gemäß Stand der Technik, also einfaches Abführen von Borverunreinigungen mit einer Menge an Chlorsilan.

Dabei wurden die Kolonnenparameter so gewählt, dass reines DCS über Kopf destillierte, während das TCS über die Blase der Kolonne abgezogen wurde.

Das so destillierte TCS enthielt noch 280 ppbw Borverbindungen.

Es zeigte sich, dass die Bor-Verbindungen nicht vollständig zusammen mit DCS aus dem Trichlorsilan abgetrennt werden können.

### Vergleichsbeispiel 2

Der Chlorsilanstrom **3** wurde in einer nachfolgenden Kolonne **6** aufdestilliert. Dies erfolgte wiederum gemäß Stand der Technik, also einfaches Abführen von Borverunreinigungen mit einer Menge an Chlorsilan.

Die Kolonnenparameter wurden so eingestellt, dass eine Mischung von 10 % DCS und 90 % TCS über Kopf abgezogen wurde.

Das der Blase entnommene TCS enthielt noch 14 ppbw Bor.

Bezogen auf 860 kg eingesetztes TCS entstanden auf diese Weise allerdings 27 kg/h TCS-Verluste.

### Beispiel 3

Die Niedersieder-haltige TCS-Fraktion **3** wurde in Kolonne **6** aufdestilliert, dabei wurde die Kopfabnahme so gewählt, dass sich eine DCS-Konzentration von 10 % im Kopfprodukt **6a** einstellte.

In **6a** wurde eine Bor-Konzentration von 88 ppm gefunden, das Blasenprodukt dieser Kolonne **6b** enthielt 17 ppbw Bor.

In der beschriebenen Weise gelingt es, mehr als 99 % der niedrig siedenden Bor-Verbindungen über das Kopfprodukt abzutrennen.

Die DCS-haltige Fraktion **6b** wurde in Kolonne **7** bei einem Überdruck von 0.1 bis 2.5 bar destilliert.

Im Blasenprodukt **7b** wurde reines TCS mit < 10 ppm DCS und 2,6 ppm Bor erhalten. Dieses Produkt wurde in die Kolonne **2** zurückgeführt.

Im Kopfprodukt **7a** wurden 99,4 % DCS, 0,6 % Monochlorsilan, sowie 770 ppm Bor gefunden.

Nach Abtrennung aller Verunreinigungen konnten aus dem Chlorsilangemisch **1** etwa 83 % reines TCS mit weniger als 20 ppb Bor erzeugt werden.

Durch die Rückführung der TCS-Fraktion **7b** erhöhte sich die Ausbeute auf 86 %.

Hinzu kommen eine STC-Fraktion **5a,** mit ca. 13% bezogen auf die Menge der Ausgangsmischung und einem Bor-Gehalt von 1 ppm und eine DCS-Fraktion **7a,** mit 0,3 % bezogen auf die Menge der Ausgangsmischung mit ca. 770 ppm Bor.

### Beispiel 4

Die Niedersieder-haltige TCS-Fraktion **3** wurde wie in Beispiel 3 beschrieben destilliert.

Allerdings wurde dem DCS-haltigen Feedstrom **6a** zur Kolonne 7 noch 20 m³/h Stickstoff **6c** mit einer Restfeuchte von kleiner als 1ppmv H₂O zugeführt.

Als Inertgase hätten auch Ar oder H₂ verwendet werden können.

Die Position der Einspeisung von Inertgas kann entweder im Zustrom oder an der Kolonne selbst erfolgen. Für das Beispiel wurde das Inertgas im Zustrom eingeschleust.

Zusätzlich wurde auf eine Tieftemperaturkondensation der des Abgases verzichtet.

Durch die Inert-Gas-Zuspeisung erhöhte sich die Abgasmenge der Kolonne.

Im Kopfprodukt **7a** wurden lediglich noch 400 ppm Bor gefunden.

Theoretisch wäre mehr als die doppelte Bormenge zu erwarten gewesen, d.h. mehr als 50 % des zur Kolonne zugeführten BCl₃ reichern sich im Abgasstrom **7c** an.

Dieser Abgasstrom, hauptsächlich Stickstoff mit Spuren BCl₃, MCS und DCS, wurde zu einem Wäscher geleitet und entsorgt.

Das in diesem Beispiel erzeugte TCS **6b** enthielt nur noch 12 ppbw Bor.

Die Ergebnisse sind in **Tabelle 1** zusammengefasst.

**Tabelle 1**

| | TCS-Ausbeute | B-Gehalt in TCS |
|---|---|---|
| Vergleichsbeispiel 1 | 96,5% | 14 ppbw |
| Vergleichsbeispiel 2 | 100 % | 280 ppbw |
| Beispiel 3 | 100 % | 17 ppbw |
| Beispiel 4 | 100 % | 12 ppbw |

### Beispiel 5

Die Niedersieder-haltige TCS-Fraktion **3** wurde wie in Beispiel 3 beschrieben destilliert.

Hierbei wurde das Solekondensat der Kolonne 6 zu Kolonne 7 ausgeschleust (vgl. Fig. 2).

Überraschenderweise verursacht diese Maßnahme noch eine weitere drastische Reduktion der Konzentrationen an Verunreinigungen, ohne dabei die TCS-Ausbeute zu reduzieren.

Offenbar beeinflusst diese Maßnahme nicht nur die Borkonzentration sondern auch die Phosphorkonzentration positiv.

Die Ergebnisse sind in **Tabelle 2** zusammengefasst.

In Beispiel 5 zeigen sich deutliche Verbesserungen gegenüber Beispiel 3, sowohl bzgl. der Bor- als auch der Phosphorverunreinigungen bei konstanter TCS-Ausbeute.

**Tabelle 2**

| | TCS-Ausbeute | Bor | Phosphor |
|---|---|---|---|
| Beispiel 3 | 100 % | 17 ppbw | 16, 2 ppba |
| Beispiel 5 | 100 % | < 5 ppbw | 3,1 ppba |

## Patentansprüche

1. Verfahren zur destillativen Reinigung von Chlorsilanen, umfassend Bereitstellen einer borhaltigen Mischung aus Chlorsilanen enthaltend TCS, DCS und STC und destillative Reinigung der Mischung aus Chlorsilanen in mehreren Destillationskolonnen,**dadurch gekennzeichnet, dass** die bereitgestellte Mischung aus Chlorsilanen einer Trennkolonne (2) zugeführt wird, deren Kolonnenparameter so gewählt werden, dass in einer ersten Fraktion (3) aus Trennkolonne (2) weniger als 10 ppm STC und in einer zweiten Fraktion (4) aus Trennkolonne (2) weniger als 10 ppm TCS enthalten sind, wobei Fraktion (3) aus Trennkolonne (2) einer dritten Kolonne (6) zugeführt und destillativ in einen Sumpfstrom (6b), der TCS enthält, und einen borangereicherten Kopfstrom (6a), der neben TCS Niedersieder wie DCS enthält, getrennt wird und Fraktion (4) aus Trennkolonne (2) einer zweiten Kolonne (5) zugeführt und destillativ in einen Kopfstrom enthaltend STC (5a) und einen borangereicherten Sumpfstrom enthaltend Hochsieder (5b) getrennt wird, um niedrig-siedende Borverbindungen mittels Kopfströmen enthaltend borangereichertes DCS und höher-siedende Borverbindungen mittels eines borangereicherten Sumpfstromes enthaltend Hochsieder aus den Destillationskolonnen abzuzweigen.

2. Verfahren nach Anspruch 1, wobei die bereit gestellte Mischung aus Chlorsilanen mittels Reaktion von metallurgischem Silicium mit HCl in einem Wirbelschichtreaktor bei 350-400 °C erzeugt wird.

3. Verfahren nach Anspruch 1 oder 2 wobei Kopfstrom (6a) aus der dritten Kolonne (6) in eine vierte Kolonne (7) geführt wird, wobei Inertgas eingespeist wird, wobei ein Kopfstrom (7a) aus der vierten Kolonne (7) enthaltend borangereichertes DCS ausgeschleust wird, ein Sumpfstrom (7b) aus der vierten Kolonne (7) der Trennkolonne (2) wiederzugeführt wird und ein Nebenstrom (7c) enthaltend Abgas aus der vierten Kolonne (7) entsorgt wird.

4. Verfahren nach Anspruch **3**, wobei jene vierte Kolonne (7) in Überdruck betrieben wird.

5. Verfahren nach Anspruch **3** oder **4**, wobei Kopfstrom (6a) aus der dritten Kolonne (6) vor Zuführen in die vierte Kolonne (7) verflüssigt wird.

6. Verfahren nach Anspruch **5**, wobei Kopfstrom (6a) mit einem Wasserkühler 6w auf eine Temperatur von etwa 10-30 °C gekühlt, das sich bildende Kondensat (6wk) in die dritte Kolonne (6) zurückgeführt, die nicht kondensierten Anteile (6wnk) einem Solekühler (6s) zugeführt werden, der den Produktstrom auf etwa -7 °C kühlt, wobei die im Solekühler (6s) nicht kondensierten Anteile (6snk) einer Tieftemperaturkühlung (6t) zugeführt und dort zu einem Kondensat (6tk) kondensiert werden, wobei das Kondensat (6tk) sowie ein Kondensat (6sk) aus dem Solekühler der vierten Kolonne (7) zugeführt werden.

## Claims

1. Process for purifying chlorosilanes by distillation, which comprises providing a boron-containing mixture of chlorosilanes containing TCS, DCS and STC and purifying the mixture of chlorosilanes by distillation in a plurality of distillation columns, **characterized in that** the mixture of chlorosilanes which is provided is fed to a separation column (2) in which the column parameters are selected so that less than 10 ppm of STC are present in a first fraction (3) from separation column (2) and less than 10 ppm of TCS are present in a second fraction (4) from separation column (2), where fraction (3) from separation column (2) is fed to a third column (6) and separated by distillation into a bottom stream (6b) containing TCS and a boron-enriched overhead stream (6a) containing TCS together with low boilers such as DCS and fraction (4) from separation column (2) is fed to a second column (5) and separated by distillation into an overhead stream containing STC (5a) and a boron-enriched bottom stream containing high boilers (5b), in order to branch off low-boiling boron compounds from the distillation columns by means of overhead streams containing boron-enriched DCS and to branch off high-boiling boron compounds by means of a boron-enriched bottom stream containing high boilers.

2. Process according to Claim 1, wherein the mixture of chlorosilanes which is provided is produced by reaction of metallurgical silicon with HCl in a fluidized-bed reactor at 350-400°C.

3. Process according to Claim 1 or 2, wherein overhead stream (6a) from the third column (6) is fed into a fourth column (7) into which inert gas is fed, with an overhead stream (7a) containing boron-enriched DCS from the fourth column (7) being discharged, a bottom stream (7b) from the fourth column (7) being recirculated to the separation column (2) and a secondary stream (7c) containing offgas from the fourth column (7) being disposed of.

4. Process according to Claim 3, wherein the fourth column (7) is operated under superatmospheric pressure.

5. Process according to Claim 3 or 4, wherein overhead stream (6a) from the third column (6) is liquefied before being fed into the fourth column (7).

6. Process according to Claim 5, wherein overhead stream (6a) is cooled by means of a water cooler 6w to a temperature of about 10-30°C, the condensate (6wk) formed is recirculated to the third column (6), the uncondensed material (6wnk) is fed to a brine cooler (6s) which cools the product stream to about -7°C, where the material (6snk) which is not condensed in the brine cooler (6s) is fed to a low-temperature cooling stage (6t) and condensed there to form a condensate (6tk) and the condensate (6tk) and a condensate (6sk) from the brine cooler are fed to the fourth column (7).

## Revendications

1. Procédé de purification distillative de chlorosilanes, comprenant la préparation d'un mélange de chlorosilanes contenant du bore contenant du TCS, du DCS et du STC et la purification distillative du mélange de chlorosilanes dans plusieurs colonnes de distillation, **caractérisé en ce que** le mélange de chlorosilanes préparé est introduit dans une colonne de séparation (2) dont les paramètres de colonne sont choisis de manière à ce que moins de 10 ppm de STC soient contenus dans une première fraction (3) de la colonne de séparation (2) et à ce que moins de 10 ppm de TCS soient contenus dans une deuxième fraction (4) de la colonne de séparation (2), la fraction (3) de la colonne de séparation (2) étant introduite dans une troisième colonne (6) et séparée par distillation en un courant de fond (6b), qui contient du TCS, et un courant de tête enrichi en bore (6a), qui, outre le TCS, contient également des composés de point d'ébullition bas tels que le DCS, et la fraction (4) de la colonne de séparation (2) étant introduite dans une deuxième colonne (5) et séparée par distillation en un courant de tête contenant du STC (5a) et un courant de fond enrichi en bore contenant des composés de point d'ébullition élevé (5b), afin de dériver des colonnes de distillation des composés de bore de point d'ébullition bas au moyen de courants de tête contenant du DCS enrichi en bore et des composés de bore de point d'ébullition élevé au moyen d'un courant de fond enrichi en bore contenant des composés de point d'ébullition élevé.

2. Procédé selon la revendication 1, dans lequel le mélange de chlorosilanes préparé est formé par réaction de silicium métallurgique avec HCl dans un réacteur à lit fluidisé à une température de 350 à 400 °C.

3. Procédé selon la revendication 1 ou 2, dans lequel le courant de tête (6a) de la troisième colonne (6) est introduit dans une quatrième colonne (7), un gaz inerte étant introduit, un courant de tête (7a) contenant du DCS enrichi en bore étant déchargé de la quatrième colonne (7), un courant de fond (7b) de la quatrième colonne (7) étant réintroduit dans la colonne de séparation (2) et un courant latéral (7c) contenant un gaz d'échappement de la quatrième colonne (7) étant éliminé.

4. Procédé selon la revendication 3, dans lequel la quatrième colonne (7) est exploitée en surpression.

5. Procédé selon la revendication 3 ou 4, dans lequel le courant de tête (6a) de la troisième colonne (6) est liquéfié avant l'introduction dans la quatrième colonne (7).

6. Procédé selon la revendication 5, dans lequel le courant de tête (6a) est refroidi par un refroidisseur à eau 6w à une température d'environ 10 à 30 °C, le condensat (6wk) qui se forme est recyclé dans la troisième colonne (6), les fractions non condensées (6wnk) sont introduites dans un refroidisseur à sol (6s) qui refroidit le courant de produit à environ -7 °C, les fractions (6snk) non condensées dans le refroidisseur à sol (6s) étant introduites dans une réfrigération cryogénique (6t) et y étant condensées en un condensat (6tk), le condensat (6tk) et un condensat (6sk) issu du refroidisseur à sol étant introduits dans la quatrième colonne (7).
